# EUROPEAN PATENT SPECIFICATION

(11) **EP 2 824 075 B1**
(45) Date of publication and mention of the grant of the patent: **11.10.2017**
(21) Application number: 13757151.9
(22) Date of filing: 04.03.2013
(51) Int. Cl.: C22B 7/00

(54) **METHOD FOR PREPARING HIGH-PURITY COBALT NITRATE CRYSTALS FROM CO/SIO2 WASTE CATALYSTS**
VERFAHREN ZUR HERSTELLUNG HOCHREINER KOBALTNITRATKRISTALLE AUS CO/SIO2-ABFALLKATALYSATOREN
PROCÉDÉ DE PRÉPARATION DE CRISTAUX DE NITRATE DE COBALT DE GRANDE PURETÉ À PARTIR DE CATALYSEURS DE DÉCHETS CO/SIO2

(30) Priority: 05.03.2012 CN 201210055799
(43) Date of publication of application: 14.01.2015
(73) Proprietor: Wuhan Kaidi Engineering Technology Research Institute Co., Ltd., Wuhan, Hubei 430223 (CN)
(72) Inventor: HAN, Yiming, Wuhan Hubei 430212 (CN); LIU, Qianqian, Wuhan Hubei 430212 (CN); LAI, Bo, Wuhan Hubei 430212 (CN); XU, Li, Wuhan Hubei 430212 (CN); SONG, Dechen, Wuhan Hubei 430212 (CN)
(74) Representative: Zeuner Summerer Stütz
(86) International application number: PCT/CN2013/072109
(87) International publication number: WO 2013/131452

(56) References cited:
- EP-A1- 2 824 200
- CN-A- 1 344 682
- CN-A- 1 401 427
- CN-A- 101 270 420
- CN-A- 101 698 152
- CN-A- 101 700 913
- CN-A- 101 700 913
- US-A1- 2006 251 563

## Description

### FIELD OF THE INVENTION

The invention relates to a method for preparation of high purity crystals of cobalt nitrate from a spent catalyst of Co/SiO₂

### BACKGROUND OF THE INVENTION

With the increasingly serious environmental pollution and the gradual depletion of oil resources, the development of non-polluting, renewable energy has become a new focus of research. Biomass is the most abundant and cheapest renewable resource, the global yield of lignocelluloses biomass produced from the photosynthesis reaches 150 billion tons each year, of which, approximately 90% have not been utilized by humans. The biomass is used to produce syngas, and the syngas is transformed into synthetic oil by Fischer-Tropsch synthesis reaction. The synthetic oil is conducted with subsequent process and distillation to obtain super clean gasoline and diesel oil that are superior to the European V standard.

Silica supported cobalt-based Co/SiO₂ catalyst is highly active in catalytic hydrogenation. The reaction rate is not affected by the water pressure, carbon is not prone to be accumulated and intoxication is avoided. The catalyst has low selectivity to CO₂ and high selectivity to long chain hydrocarbon, and the products contain a relatively small amount of oxygenated compounds. Thus, the silica supported cobalt-based C_{O}/SiO₂catalyst is a relatively good and extensively studied catalyst system. The cobalt mineral resource in China is very scarce, and the currently detected exploitation quantity is less than 2% of the total exploitation quantity of the world, but the usage thereof in China each year accounts for 25% of the total consumption of the world. Thus, the cobalt mineral resource in China is seriously relied on import, which results in high price and further leading to high production costs of the catalyst. Cobalt is recovered from a spent catalyst in the cobalt-based Fischer-Tropsch synthesis reaction to reproduce the cobalt-based catalyst, which not only decreases the environmental pollution of the waste catalyst and improves the utilization of the cobalt resources, but also largely decreases the production cost of the cobalt catalyst, thereby being environmental friendly and having economic benefit.

Chinese Patent Application No. 200810055107.0 published as CN 101270420 A,has disclosed a method for recovering cobalt from cobalt-based Fischer-Tropsch synthesis catalyst, and the method includes: adding deionized water and the spent cobalt-based Fischer-Tropsch catalyst (spent cobalt-based catalyst containing carriers of SiO₂, Al₂O₃, ZrO₂, and TiO₂) to a reactor in a weight ratio of 1-5:1, introducing CO into the reactor, and heating the reactor to a constant temperature to carry out the reaction; decreasing the temperature and discharging CO from the reactor, and discharging a liquid containing cobalt from the reactor; adding an alkali solution to the liquid containing cobalt to allow the cobalt to precipitate in the form of Co(OH)₂; dissolving the precipitate by adding nitric acid, and evaporating a resulting solution to crystallize CO(NO₃)_{2.6}H2O. In the above method, Co(OH)₂ is first precipitated and is then dissolved by nitric acid to obtain CO(NO₃)_{2.6}H₂O, and the purity of Co(NO₃)₂.₆H₂O is only 98% below. Chinese Patent Application No. 200910272794.6 published as CN101700913 A, discloses a method for preparing highly purified cobalt nitrate using a alumina supported cobalt based spent catalyst in Fischer-Tropsch synthesis, and the method includes: grinding, dissolving by concentrated hydrochloric acid, precipitating cobalt by sodium sulfide, precipitating cobalt by dicarboxyl, calcinating, dissolving by nitric acid, and evaporating and crystallizing. A final product Co(NO₃)₂.₆H₂O has a purity of 99% above. However, an intermediate CoS produced in the process of reproduction and recover of the spent catalyst has fine granules, which is difficult to filter and easily results in cobalt loss, thereby decreasing the recovery rate of cobalt to approximately 92%.

### SUMMARY OF THE INVENTION

In view of the above-described problems, it is one objective of the invention to provide a method for preparing high purity of a crystal of cobalt nitrate from a spent catalyst of Co/SiO₂.The method features a high recovery rate and high product purity.

To achieve the above objective, in accordance with one embodiment of the invention, there is provided a method for preparing a crystal of cobalt nitrate from a spent catalyst of Co/SiO₂, the method comprises the following steps
1) calcining a spent catalyst of Co/SiO₂ in the presence of air for between 3 and 6 hours at a temperature of between 350 and 500°C, cooling to room temperature, and grinding the spent catalyst of Co/SiO₂ to yield a powder;
2) introducing the powder to a fluidized bed reactor, allowing the powder to be reduced for between 8 and 12 hours in the presence of a mixed gas of H2 and N₂, a volume ratio of H₂ and N₂ in the mixed gas being between 1: 1 and 4: 1;
3) adding a reduced spent catalyst to an excessive dilute nitric acid solution for dissolution, filtering, and collecting a cobalt nitrate solution;
4) adjusting a pH value of the cobalt nitrate solution using an alkali solution to be 1.5, adding a preheated oxalic acid solution having a temperature of between 25 and 80°C and a pH value of 1.5 to the cobalt nitrate solution in the presence of a water bath of between 25 and 80°C, adjusting a pH value of a resulting solution using a dilute alkali solution to be 1.5, immediately filtering the resulting solution to yield a precipitate of cobalt oxalate, washing the precipitate of cobalt oxalate using deionized water to yield a neutral filtrate, wherein, a mole number of oxalic acid in the oxalic acid solution is controlled to be between 2 and 3 times a mole number of cobalt in the cobalt nitrate solution;
5) drying the precipitate of cobalt oxalate and calcining for between 4 and 8 hours at a temperature of between 550 and 650°C, to yield cobalt oxide;
6) dissolving the cobalt oxide with a dilute nitric acid solution to yield a second cobalt nitrate solution; and
7) evaporating and crystallizing the second cobalt nitrate solution to yield a crystal of Co(NO₃)₂·6H₂O.

In step 2), a flow rate of the mixed gas is between 1000 and 4000 h⁻¹, a pressure of the fluidized bed reactor is between 0.1 and 1 MPa, and a reaction temperature is between 350 and 750°C.

In step 3), the dilute nitric acid solution has a concentration of 1 and 3 mol/L.

In step 6), the dilute nitric acid solution has a concentration of 1 and 3 mol/L.

In step 4), the water bath has a temperature of 70°C, the oxalic acid solution is preheated to 70°C, and the deionized water is preheated to 70°C.

Advantages of the present disclosure are summarized as follows. The invention employs a spent catalyst of Co/SiO₂ from Fischer-Tropsch process to prepare a high purity of a crystal of cobalt nitrate. Heavy hydrocarbons on the surface of the catalyst are removed through aerobic calcination. The spent catalyst is reduced by a reducing mixed gas comprising more than 50 vol. % of hydrogen to yield cobalt. The reduction product is dissolved in the excessive dilute nitric acid to yield the cobalt nitrate solution, which is allowed to react with an oxalic acid solution to produce a precipitate of cobalt oxalate. The oxalic acid solution is excessively added so as to facilitate the precipitation of cobalt thereby improving the conversion of cobalt. In addition, the pH value is controlled at 1.5, to ensure the complete precipitation of cobalt to form cobalt oxalate. The temperature is controlled at between 25 and 80°C, particularly at 70°C. Too much low temperature results in the obtained cobalt oxalate granules are too much fine, which cause a great loss in the next filtering and washing. In the process of precipitating cobalt oxalate, the released hydrogen ions decreases the pH value of the mixture, so, for the benefit of the complete precipitation of cobalt, the pH value at the reaction end should be adjusted to be 1.5. Thereafter, the cobalt oxalate is decomposed under high temperature to yield cobalt oxide, and the latter is added to dilute nitric acid to yield a solution of cobalt nitrate. The solution is evaporated and crystallized to yield high purity of a crystal of C_{O}(NO₃)₂6H₂O.

The process conditions are strictly controlled in the present disclosure, so that the resulting product has high recovery rate and high purity, and can be directly used for the industrial applications. The method of the present disclosure has simple operation, short flow path, and low requirements on devices and process condition, and thus is suitable for large-scale applications.

### DETAILED DESCRIPTION OF THE EMBODIMENTS

For further illustrating the invention, experiments detailing a method for preparing a crystal of cobalt nitrate from a spent catalyst of Co/SiO₂ are described below. It should be noted that the following examples are intended to describe and not to limit the invention.

### Example 1

1) 20.01 g of a spent catalyst of Co/SiO₂ comprising 12.1 wt. % of cobalt was collected. The spent catalyst was added to a muffle furnace, air was charged, and the temperature was increased from room temperature to 350°C, and was allowed to keep for 6 hours for removal of heavy hydrocarbons on the surface of the catalyst. The muffle furnace was cooled to room temperature and a resulting product was uniformly ground to yield a powder.
2) The powder was introduced to a fluidized bed reactor, and reduced in the presence of a mixed gas of H₂ and N₂ (a volume ratio of H₂ and N₂ in the mixed gas was 1: 1) at 400°C and 1 MPa for 10 hours, and the flow rate of the mixed gas is 4000 h⁻¹.
3) The reduced spent catalyst was added to 200 mL of a dilute nitric acid solution having a concentration of 3 mol/L. The mixture was stirred, and filtered to collect a filtrate being a cobalt nitrate solution.
4) The pH value of the cobalt nitrate solution was adjusted to be 1.5by saturated NaOH solution. 12.95 g of an oxalic acid solid (H₂C₂O₄·2H₂O) was dissolved in deionized water, and the pH value thereof was adjusted to be 1.5 using 1 wt. % of NaOH solution, whereby yielding an oxalic acid solution. The oxalic acid solution was preheated to 70°C, added drop wise (0.01 mL/s) to the cobalt nitrate solution in the presence of a water bath of 70°C, and stirred for 15 min. The pH value of a resulting solution was adjusted to be 1.5 using 5 wt. % NaOH solution, and the solution was filtered immediately to yield a precipitate of CoC₂O₄. The precipitate of CoC₂O₄ was washed with deionized water (70°C) to yield a neutral filtrate.
5) The precipitate of CoC₂O₄ was dried and calcined in a muffle furnace at 550°C for 5 hours to yield cobalt oxide (Co₂O₃).
6) The cobalt oxide was dissolved in 3 mol/L of a nitric acid solution to yield a second cobalt nitrate solution.
7) The second cobalt nitrate solution was evaporated in a 70°C water bath. Crystalline films were constantly formed and collected. The crystalline films were dried to a constant weight to yield 11.57 g of Co(NO₃)_{2·}6H₂O.

According to the measurement method provided in GBT 15898-1995, the purity of the product of Co(NO₃)_{2·}6H₂O was 99.40%, and the recovery percent of cobalt in this example was 96.18%.

### Example 2

1) 10.24 g of a spent catalyst of Co/SiO₂comprising 18.36 wt. % of cobalt was collected. The spent catalyst was added to a muffle furnace, air was charged, and the temperature was increased from room temperature to 400°C, and was allowed to keep for 4 hours for removal of heavy hydrocarbons on the surface of the catalyst. The muffle furnace was cooled to room temperature and a resulting product was uniformly ground to yield a powder.
2) The powder was introduced to a fluidized bed reactor, and reduced in the presence of a mixed gas of H₂ and N₂ (a volume ratio of H2 and N₂ in the mixed gas was3: 1) at 750°C and 0.5 MPa for 8 hours, and the flow rate of the mixed gas is 3000 h⁻¹.
3) The reduced spent catalyst was added to 160 mL of a dilute nitric acid solution having a concentration of 2 mol/L. The mixture was stirred, and filtered to collect a filtrate being a cobalt nitrate solution.
4) The pH value of the cobalt nitrate solution was adjusted to be 1.5 by saturated NaOH solution. 10.05 g of an oxalic acid solid (H₂C₂O₄-2H₂O) was dissolved in deionized water, and the pH value thereof was adjusted to be 1.5 using 1 wt. % of NaOH solution, whereby yielding an oxalic acid solution. The oxalic acid solution was preheated to 70°C, added drop wise (0.01 mL/s) to the cobalt nitrate solution in the presence of a water bath of 70°C, and stirred for 15 min. The pH value of a resulting solution was adjusted to be 1.5 using 5 wt. % NaOH solution, and the solution was filtered immediately to yield a precipitate of CoC₂O₄. The precipitate of CoC₂O₄ was washed with deionized water (70°C) to yield a neutral filtrate.
5) The precipitate of CoC₂O₄ was dried and calcined in a muffle furnace at 650°C for 4 hours to yield cobalt oxide (Co₂O₃).
6) The cobalt oxide was dissolved in 3 mol/L of a nitric acid solution to yield a second cobalt nitrate solution.
7) The second cobalt nitrate solution was evaporated in a 70°C water bath. Crystalline films were constantly formed and collected. The crystalline films were dried to a constant weight to yield 9.04 g of Co(NO₃)₂-6H₂O.

According to the measurement method provided in GBT 15898-1995, the purity of the product of Co(NO₃)₂-6H₂O was 99.57%, and the recovery percent of cobalt in this example was 96.94%.

### Example 3

1) 15.62 g of a spent catalyst of Co/SiO₂comprising 22.64 wt. % of cobalt was collected. The spent catalyst was added to a muffle furnace, air was charged, and the temperature was increased from room temperature to 500°C, and was allowed to keep for 3 hours for removal of heavy hydrocarbons on the surface of the catalyst. The muffle furnace was cooled to room temperature and a resulting product was uniformly ground to yield a powder.
2) The powder was introduced to a fluidized bed reactor, and reduced in the presence of a mixed gas of H₂ and N₂ (a volume ratio of H₂ and N₂ in the mixed gas was4: 1) at 600°C and 0.1 MPa for 12 hours, and the flow rate of the mixed gas is 1000 h⁻¹.
3) The reduced spent catalyst was added to 300 mL of a dilute nitric acid solution having a concentration of 3 mol/L. The mixture was stirred, and filtered to collect a filtrate being a cobalt nitrate solution.
4) The pH value of the cobalt nitrate solution was adjusted to be 1.5 by saturated NaOH solution. 18.91 g of an oxalic acid solid (H₂C₂O₄-2H₂O) was dissolved in deionized water, and the pH value thereof was adjusted to be 1.5 using 1 wt. % of NaOH solution, whereby yielding an oxalic acid solution. The oxalic acid solution was preheated to 70°C, added dropwise (0.01 mL/s) to the cobalt nitrate solution in the presence of a water bath of 70°C, and stirred for 15 min. The pH value of a resulting solution was adjusted to be 1.5 using 5 wt. % NaOH solution, and the solution was filtered immediately to yield a precipitate of CoC₂O₄. The precipitate of CoC₂O₄ was washed with deionized water (70°C) to yield a neutral filtrate.
5) The precipitate of CoC₂O₄ was dried and calcined in a muffle furnace at 600°C for 8 hours to yield cobalt oxide (Co₂O₃).
6) The cobalt oxide was dissolved in 1 mol/L of a nitric acid solution to yield a second cobalt nitrate solution.
7) The second cobalt nitrate solution was evaporated in a 70°C water bath. Crystalline films were constantly formed and collected. The crystalline films were dried to a constant weight to yield 16.94 g of Co(NO₃)₂·6H₂O.

According to the measurement method provided in GBT 15898-1995, the purity of the product of Co(NO₃)₂·6H₂O was 99.63%, and the recovery percent of cobalt in this example was 96.64%.

### Example 4

1) 15.62 g of a spent catalyst of Co/SiO₂comprising 22.64 wt. % of cobalt was collected. The spent catalyst was added to a muffle furnace, air was charged, and the temperature was increased from room temperature to 450°C, and was allowed to keep for 5 hours for removal of heavy hydrocarbons on the surface of the catalyst. The muffle furnace was cooled to room temperature and a resulting product was uniformly ground to yield a powder.
2) The powder was introduced to a fluidized bed reactor, and reduced in the presence of a mixed gas of H₂ and N₂ (a volume ratio of H₂ and N₂ in the mixed gas was3: 1) at 350°C and 0.3 MPa for 10 hours, and the flow rate of the mixed gas is 3000 h⁻¹.
3) The reduced spent catalyst was added to 300 mL of a dilute nitric acid solution having a concentration of 1 mol/L. The mixture was stirred, and filtered to collect a filtrate being a cobalt nitrate solution.
4) The pH value of the cobalt nitrate solution was adjusted to be 1.5 by saturated NaOH solution. 22.12 g of an oxalic acid solid (H₂C₂O₄·2H₂O) was dissolved in deionized water, and the pH value thereof was adjusted to be 1.5 using 1 wt. % of NaOH solution, whereby yielding an oxalic acid solution. The oxalic acid solution was preheated to 80°C, added drop wise (0.01 mL/s) to the cobalt nitrate solution in the presence of a water bath of 80°C, and stirred for 15 min. The pH value of a resulting solution was adjusted to be 1.5 using 5 wt. % NaOH solution, and the solution was filtered immediately to yield a precipitate of CoC₂O₄. The precipitate of CoC₂O₄ was washed with deionized water (70°C) to yield a neutral filtrate.
5) The precipitate of CoC₂O₄ was dried and calcined in a muffle furnace at 600°C for 6 hours to yield cobalt oxide (Co₂O₃).
6) The cobalt oxide was dissolved in 1 mol/L of a nitric acid solution to yield a second cobalt nitrate solution.
7) The second cobalt nitrate solution was evaporated in a 70°C water bath. Crystalline films were constantly formed and collected. The crystalline films were dried to a constant weight to yield 17.32 g of Co(NO₃)₂·6H₂O.

According to the measurement method provided in GBT 15898-1995, the purity of the product of Co(NO₃)₂·6H₂O was 99.55%, and the recovery percent of cobalt in this example was 97.04%.

## Claims

1. A method for preparing a crystal of cobalt nitrate from a spent catalyst of Co/SiO₂, the method comprising the following steps:
1) calcining a spent catalyst of Co/SiO₂ in the presence of air for between 3 and 6 hours at a temperature of between 350 and 500°C, cooling to room temperature, and grinding the spent catalyst of Co/SiO₂ to yield a powder;
2) introducing the powder to a fluidized bed reactor, allowing the powder to be reduced for between 8 and 12 hours in the presence of a mixed gas of H₂ and N₂, a volume ratio of H₂ and N₂ in the mixed gas being between 1: 1 and 4: 1;
3) adding a reduced spent catalyst to an excessive dilute nitric acid solution for dissolution, filtering, and collecting a cobalt nitrate solution;
4) adjusting a pH value of the cobalt nitrate solution using an alkali solution to be 1.5, adding a preheated oxalic acid solution having a temperature of between 25 and 80°Cand a pH value of 1.5 to the cobalt nitrate solution in the presence of a water bath of between 25 and 80°C, adjusting a pH value of a resulting solution using a dilute alkali solution to be 1.5, immediately filtering the resulting solution to yield a precipitate of cobalt oxalate, washing the precipitate of cobalt oxalate using deionized water to yield a neutral filtrate, wherein, a mole number of oxalic acid in the oxalic acid solution is controlled to be between 2 and 3 times a mole number of cobalt in the cobalt nitrate solution;
5) drying the precipitate of cobalt oxalate and calcining for between 4 and 8 hours at a temperature of between 550 and 650°C, to yield cobalt oxide;
6) dissolving the cobalt oxide with a dilute nitric acid solution to yield a second cobalt nitrate solution; and
7) evaporating and crystallizing the second cobalt nitrate solution to yield a crystal of Co(NO₃)₂·6H₂O.

2. The method of claim 1, **characterized in that** in step 2), a flow rate of the mixed gas is between 1000 and 4000 h⁻¹, a pressure of the fluidized bed reactor is between 0.1 and 1 MPa, and a reaction temperature is between 350 and 750°C.

3. The method of claim 1 or 2, **characterized in that** in step 3), the dilute nitric acid solution has a concentration of 1 and 3 mol/L.

4. The method of claim 1 or 2, **characterized in that** in step 6), the dilute nitric acid solution has a concentration of 1 and 3 mol/L.

5. The method of claim 3, **characterized in that** in step 6), the dilute nitric acid solution has a concentration of 1 and 3 mol/L.

6. The method of claim 1 or 2, **characterized in that** in step 4), the water bath has a temperature of 70°C, the oxalic acid solution is preheated to 70°C, and the deionized water is preheated to 70°C.

7. The method of claim 3, **characterized in that** in step 4), the water bath has a temperature of 70°C, the oxalic acid solution is preheated to 70°C, and the deionized water is preheated to 70°C.

8. The method of claim 4, **characterized in that** in step 4), the water bath has a temperature of 70°C, the oxalic acid solution is preheated to 70°C, and the deionized water is preheated to 70°C.

9. The method of claim 5, wherein in step 4), the water bath has a temperature of 70°C, the oxalic acid solution is preheated to 70°C, and the deionized water is preheated to 70°C.

## Patentansprüche

1. Verfahren zur Herstellung eines Cobaltnitratkristalls aus Co/SiO₂-Abfallkatalysatoren, wobei das Verfahren die folgenden Schritte umfasst:
1) Calcinieren eines Co/SiO₂-Abfallkatalysators in Gegenwart von Luft über einen Zeitraum von 3 bis 6 Stunden bei einer Temperatur zwischen 350 und 500°C, Abkühlen auf Zimmertemperatur und Mahlen des Co/SiO₂-Abfallkatalysators, um ein Pulver zu erhalten;
2) Einleiten des Pulvers in einen Wirbelschichtreaktor, wobei das Pulver über einen Zeitraum von 8 bis 12 Stunden in Gegenwart eines Gasgemischs aus H₂ und N₂, wobei das Volumenverhältnis von H₂ zu N₂ in dem Gasgemisch zwischen 1:1 und 4:1 liegt, reduzieren gelassen wird;
3) Zugeben eines reduzierten Abfallkatalysators zu einer übermäßig verdünnten Salpetersäurelösung zum Lösen, Filtrieren und Sammeln einer Cobaltnitratlösung;
4) Einstellen eines pH-Werts der Cobaltnitratlösung auf 1,5 unter Verwendung einer Alkalilösung, Zugeben einer vorerhitzten Oxalsäurelösung mit einer Temperatur zwischen 25 und 80°C und einem pH-Wert von 1,5 zu der Cobaltnitratlösung in Gegenwart eines Wasserbads zwischen 25 und 80°C, Einstellen eines pH-Werts einer so erhaltenen Lösung unter Verwendung einer verdünnten Alkalilösung auf 1,5, unmittelbares Filtrieren der so erhaltenen Lösung, um eine Ausfällung von Cobaltoxalat zu erhalten, Waschen der Ausfällung von Cobaltoxalat unter Verwendung von entionisiertem Wasser, um ein neutrales Filtrat zu erhalten, wobei eine Molzahl von Oxalsäure in der Oxalsäurelösung so eingestellt wird, dass sie das Zweifache bis Dreifache einer Molzahl von Cobalt in der Cobaltnitratlösung beträgt;
5) Trocknen der Ausfällung des Cobaltoxalats und Calcinieren über einen Zeitraum von 4 bis 8 Stunden bei einer Temperatur von 550 bis 650 °C, um Cobaltoxid zu erhalten;
6) Lösen des Cobaltoxids mit einer verdünnten Salpetersäurelösung, um eine zweite Cobaltnitratlösung zu erhalten; und
7) Verdampfen und Kristallisieren der zweiten Cobaltnitratlösung, um ein Kristall von Co(NO₃)_{2·}6H₂O zu erhalten.

2. Verfahren nach Anspruch 1, **dadurch gekennzeichnet, dass** in Schritt 2) eine Strömungsrate des Gasgemischs zwischen 1000 und 4000 h⁻¹ beträgt, ein Druck des Wirbelschichtreaktors zwischen 0,1 und 1 MPa beträgt und eine Reaktionstemperatur zwischen 350 und 750°C beträgt.

3. Verfahren nach Anspruch 1 oder 2, **dadurch gekennzeichnet, dass** in Schritt 3) die verdünnte Salpetersäurelösung eine Konzentration von 1 und 3 mol/l aufweist.

4. Verfahren nach Anspruch 1 oder 2, **dadurch gekennzeichnet, dass** in Schritt 6) die verdünnte Salpetersäurelösung eine Konzentration von 1 und 3 mol/l aufweist.

5. Verfahren nach Anspruch 3, **dadurch gekennzeichnet, dass** in Schritt 6) die verdünnte Salpetersäurelösung eine Konzentration von 1 und 3 mol/l aufweist.

6. Verfahren nach Anspruch 1 oder 2, **dadurch gekennzeichnet, dass** in Schritt 4) das Wasserbad eine Temperatur von 70°C aufweist, die Oxalsäurelösung auf 70°C vorerhitzt wird und das entionisierte Wasser auf 70°C vorerhitzt wird.

7. Verfahren nach Anspruch 3, **dadurch gekennzeichnet, dass** in Schritt 4) das Wasserbad eine Temperatur von 70°C aufweist, die Oxalsäurelösung auf 70°C vorerhitzt wird und das entionisierte Wasser auf 70°C vorerhitzt wird.

8. Verfahren nach Anspruch 4, **dadurch gekennzeichnet, dass** in Schritt 4) das Wasserbad eine Temperatur von 70°C aufweist, die Oxalsäurelösung auf 70°C vorerhitzt wird und das entionisierte Wasser auf 70°C vorerhitzt wird.

9. Verfahren nach Anspruch 5, wobei in Schritt 4) das Wasserbad eine Temperatur von 70°C aufweist, die Oxalsäurelösung auf 70°C vorerhitzt wird und das entionisierte Wasser auf 70°C vorerhitzt wird.

## Revendications

1. Procédé de préparation d'un cristal de nitrate de cobalt à partir d'un catalyseur usé de Co/SiO₂, le procédé comprenant les étapes suivantes consistant à :
1) calciner un catalyseur usé de Co/SiO₂ en présence d'air pendant de 3 à 6 heures à une température comprise entre 350 et 500 °C, refroidir à température ambiante, et broyer le catalyseur usé de Co/SiO₂ pour donner une poudre ;
2) introduire la poudre dans un réacteur à lit fluidisé, permettre à la poudre d'être réduite pendant de 8 à 12 heures en présence d'un gaz mélangé de H₂ et de N₂, un rapport volumique de H₂ et N₂ dans le gaz mélangé étant compris entre 1:1 et 4:1 ;
3) ajouter un catalyseur usé réduit à une solution d'acide nitrique dilué en excès pour une dissolution, filtrer, et recueillir une solution de nitrate de cobalt ;
4) ajuster une valeur de pH de la solution de nitrate de cobalt en utilisant une solution alcaline à 1,5, ajouter une solution d'acide oxalique préchauffée possédant une température comprise entre 25 et 80 °C et une valeur de pH de 1,5 à la solution de nitrate de cobalt en présence d'un bain-marie de température comprise entre 25 et 80 °C, ajuster une valeur de pH d'une solution résultante en utilisant une solution alcaline diluée pour être 1,5, filtrer immédiatement la solution résultante pour donner un précipité d'oxalate de cobalt, laver le précipité d'oxalate de cobalt en utilisant de l'eau désionisée pour donner un filtrât neutre, dans lequel, un nombre molaire d'acide oxalique dans la solution d'acide oxalique est contrôlé pour être compris entre 2 et 3 fois un nombre molaire de cobalt dans la solution de nitrate de cobalt ;
5) sécher le précipité d'oxalate de cobalt et calciner pendant de 4 à 8 heures à une température comprise entre 550 et 650 °C, pour donner du dioxyde de cobalt ;
6) dissoudre l'oxyde de cobalt avec une solution d'acide nitrique est situé pour donner une seconde solution de nitrate de cobalt ; et
7) évaporer et cristalliser la seconde solution de nitrate de cobalt pour donner un cristal de Co(NO₃)₂·6H₂O.

2. Procédé selon la revendication 1, **caractérisé en ce que** lors de l'étape 2) , un débit d'écoulement du gaz mélangé est compris entre 1 000 et 4 000 h⁻¹, une pression du réacteur à lit fluidisé est comprise entre 0,1 et 1 MPa, et une température de réaction est comprise entre 350 et 750 °C.

3. Procédé selon la revendication 1 ou la revendication 2, **caractérisé en ce que** lors de l'étape 3), la solution d'acide nitrique dilué possède une concentration comprise entre 1 et 3 mol/L.

4. Procédé selon la revendication 1 ou la revendication 2, **caractérisé en ce que** lors de l'étape 6), la solution d'acide nitrique dilué possède une qu'on s'en traction comprise entre 1 et 3 mol/L.

5. Procédé selon la revendication 3, **caractérisé en ce que** lors de l'étape 6), la solution d'acide nitrique dilué possède une qu'on s'en traction comprise entre 1 et 3 mol/L.

6. Procédé selon la revendication 1 ou la revendication 2, **caractérisé en ce que** lors de l'étape 4), le bain-marie possède une température de 70 °C, la solution d'acide oxalique est préchauffée à 70 °C, et l'eau désionisée est préchauffée à 70 °C.

7. Procédé selon la revendication 3, **caractérisé en ce que** lors de l'étape 4), le bain-marie possède une température de 70 °C, la solution d'acide oxalique est préchauffée à 70 °C, et l'eau désionisée est préchauffée à 70 °C.

8. Procédé selon la revendication 4, **caractérisé en ce que** lors de l'étape 4), le bain-marie possède une température de 70 °C, la solution d'acide oxalique est préchauffée à 70 °C, et l'eau désionisée est préchauffée à 70 °C.

9. Procédé selon la revendication 5, **caractérisé en ce que** lors de l'étape 4), le bain-marie possède une température de 70 °C, la solution d'acide oxalique est préchauffée à 70 °C, et l'eau désionisée est préchauffée à 70 °C.
